# DEMANDE DE BREVET EUROPEEN

(11) **EP 2 832 254 A1**
(43) Date de publication de la demande: **04.02.2015**
(21) Numéro de dépôt: 14178398.5
(22) Date de dépôt: 24.07.2014
(51) Int. Cl.: A45D 26/00, A61B 17/30, A45D 40/18, A61B 19/02

(54) **Dispositif de pinces et son système de protection**

(30) Priorité: 01.08.2013 FR 1357644
(71) Demandeur: F.I.M.C.O., 66160 Le Boulou (FR)
(72) Inventeur: Guyard, Yves, 66160 Le Boulou (FR)
(74) Mandataire: Rhein, Alain

(57) **Abrégé**

Dispositif de pinces (1) et son système de protection (4), du type où ledit dispositif de pinces (1) comporte deux branches (2) mobiles en rapprochement et en éloignement d'un plan axial médian, au travers d'une déformation élastique, ou d'un déplacement de type pivotement, rappelées élastiquement en ouverture.

Le système de protection (4) consiste en un bloc comportant une cavité (40) destinée à loger les extrémités distales (22) des branches (2), et la cavité (4) et le dispositif de pinces (1) comportent d'une part des éléments (20,44) de formes complémentaires conçus aptes à permettre la rétention par encliquetage du dispositif de pinces (1) dans la cavité (40), tout en autorisant le maintien des branches (2) écartées en sorte que les extrémités distales (22) soient maintenues hors de contact de l'une avec l'autre ; et d'autre part des moyens (46) d'immobilisation dans le sens axial du dispositif de pinces (1) par rapport au système de protection (4).

## Description

La présente invention a pour objet un dispositif de pinces et son système de protection, plus particulièrement, mais non exclusivement, destiné au domaine des pinces chirurgicales.

Un dispositif de pinces, comporte deux branches mobiles en rapprochement et en éloignement d'un plan axial médian, au travers d'une déformation élastique ou d'un déplacement de type pivotement, rappelées élastiquement en ouverture, et en sorte que les extrémités distales desdites branches puissent saisir, maintenir, puis relâcher un objet.

Les branches des dispositifs de pinces présentent des formes variées, chacune adaptée à une utilisation particulière. Lorsqu'une certaine précision est nécessaire, particulièrement dans le domaine chirurgical, il est nécessaire que les branches ne subissent aucune déformation, ce qui justifie de les protéger.

De manière habituelle, dans le domaine chirurgical, les branches sont protégées en étant serrées l'une contre l'autre et introduites ainsi dans un tube souple, du silicone généralement, ce qui présente un inconvénient pour les opérations de nettoyage et de stérilisation, puisqu'alors ce tube doit être retiré et que les branches, et plus particulièrement leurs extrémités distales, ne sont plus protégées.

Une solution connue est décrite au travers du document US 2009/054925. Elle vise un dispositif de pinces, constituées de deux branches, reliées à une extrémité de jonction et libres à leur extrémité opposée, prévues mobiles en rapprochement et en éloignement d'un plan axial médian, par déformation élastique. Ce document vise aussi le système de protection sous forme d'un capuchon de telles pinces, ledit capuchon étant constitué d'un bloc comportant une cavité destinée à loger les extrémités distales desdites branches. En insertion, les branches sont maintenues au sein de ladite cavité par encliquetage desdites extrémités distales, les immobilisant dans le sens axial.

Un inconvénient d'un tel système de protection réside dans le fait que les extrémités distales des branches se retrouvent être au contact l'une de l'autre une fois le dispositif de pinces inséré dans le capuchon.

Une solution similaire connue du document WO 02/34081, décrivant un dispositif de pinces avec son système de protection sous forme d'un étui présentant une paroi centrale longitudinale effilée, autour et au contact de laquelle est positionnée le dispositif de pinces, par insertion depuis une extrémité dudit étui. Les branches, en particulier leur extrémité distale, viennent au contact de part et d'autre de ladite paroi médiane.

Une autre solution est décrite dans le document US 4 836 596 proposant un étui pourvu d'un ergot central, maintenant écartée les extrémités distales de branches en insertion du dispositif. Des plots situés en vis-à-vis au niveau des parois latérales intérieures de l'étui, empêchent que les branches écartées par ledit ergot ne viennent au contact desdites parois latérales. Toutefois, il existe une zone de contact, même minimisée sous forme de points, des branches avec l'ergot et les plots.

La présente invention a pour but de proposer un dispositif de pinces et son système de protection, plus particulièrement destiné au domaine chirurgical, permettant de remédier aux divers inconvénients précités.

En particulier, l'invention permet de maintenir écartées les extrémités distales des branches lorsque le dispositif de pinces est introduit et maintenu au sein de sa protection, assurant une stérilisation de l'intégralité des branches au niveau de leur surface d'utilisation, condition nécessaire dans les domaines médical et chirurgical.

Pour ce faire, le dispositif de pinces et son système de protection selon l'invention, est du type où ledit dispositif de pinces comporte deux branches mobiles en rapprochement et en éloignement d'un plan axial médian, au travers d'une déformation élastique, ou d'un déplacement de type pivotement, rappelées élastiquement en ouverture, et en sorte que les extrémités distales desdites branches puissent saisir, maintenir, puis relâcher un objet, et il se caractérise en ce que ledit système de protection consiste en un bloc comportant une cavité destinée à loger les extrémités distales desdites branches, et en ce que ladite cavité et ledit dispositif de pinces comportent d'une part des éléments de formes complémentaires conçus aptes à permettre la rétention par encliquetage dudit dispositif de pinces dans ladite cavité, tout en autorisant le maintien desdites branches écartées en sorte que lesdites extrémités distales soient maintenues hors de contact de l'une avec l'autre ; et d'autre part des moyens d'immobilisation dans le sens axial dudit dispositif de pinces par rapport audit système de protection.

Selon une caractéristique additionnelle du dispositif selon l'invention, le système de protection comporte des moyens d'immobilisation en rotation axiale du dispositif de pinces.

Selon une autre caractéristique additionnelle du dispositif selon l'invention, le système de protection est réalisé dans un matériau supportant des conditions de stérilisation.

Selon une autre caractéristique additionnelle du dispositif selon l'invention, le système de protection est réalisé dans un polymère de la famille des polyacétals.

Le dispositif de pinces et son système de protection selon l'invention peuvent ainsi subir les phases de nettoyage, décontamination et stérilisation, puis être stockés sans risques de détérioration.

Selon un mode de réalisation particulier, la cavité du système de protection consiste en une gorge pratiquée longitudinalement, ouverte à au moins une extrémité, et qui présente, dans le but de rétention, une zone de profil femelle concordant avec le profil d'au moins une partie du dispositif de pinces, l'introduction de ladite partie dans ladite zone, nécessitant de traverser une zone de moindre largeur ce qui exige de contraindre les branches en rapprochement.

Selon une caractéristique additionnelle du dispositif selon l'invention, les moyens d'immobilisation dans le sens axial consistent en des empreintes males aptes à coopérer avec des empreintes femelles concordantes que comporte le dispositif de pinces.

Selon une autre caractéristique additionnelle du dispositif selon l'invention, les empreintes mâles consistent en une ou plusieurs lames qui s'étendent transversalement à l'axe de la gorge, et partiellement dans ladite gorge depuis les parois en regard qui délimitent ladite gorge, tandis les empreintes femelles consistent en au moins une fente pratiquée perpendiculairement au plan axial médian du dispositif de pinces.

Les avantages et les caractéristiques du dispositif de pinces et son système de protection selon l'invention, ressortiront plus clairement de la description qui suit et qui se rapporte au dessin annexé, lequel en représente plusieurs modes de réalisation non limitatifs.

Dans le dessin annexé :
- la figure 1 représente une vue schématique en plan d'un dispositif de pinces et de son système de protection selon l'invention.
- la figure 2 représente une vue schématique en élévation du système de protection.
- la figure 3 représente une vue schématique en plan du même système de protection.

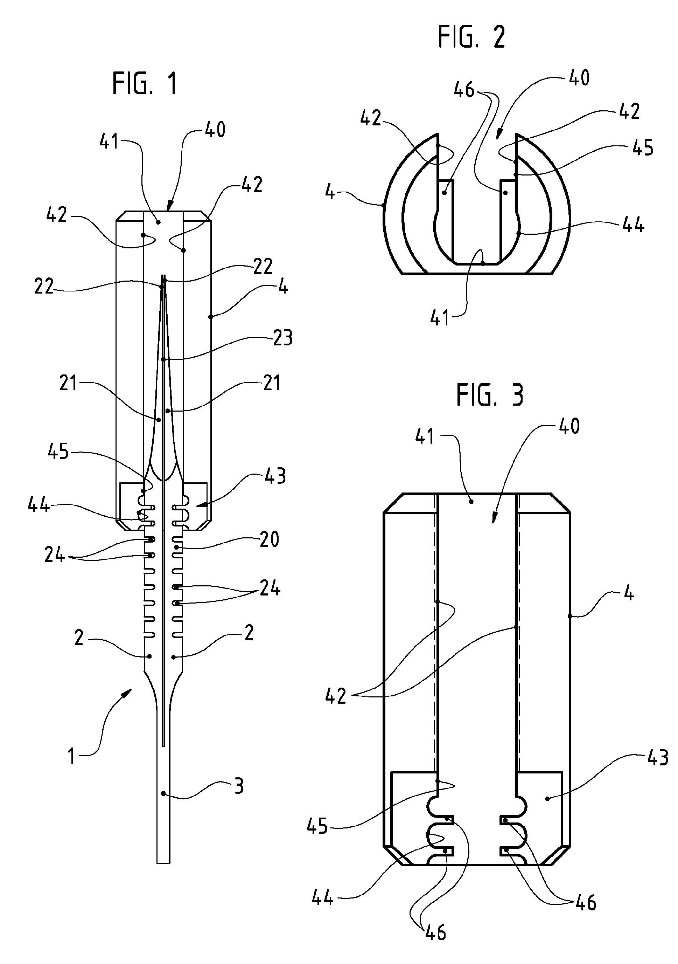

En référence à la figure 1, on peut voir, selon un mode de réalisation particulier, non limitatif, un dispositif de pinces 1 selon l'invention, qui comprend deux branches 2, réunies à une extrémité par un segment 3, et qui comportent d'une part en zone médiane un corps 20, et d'autre part deux segments libres 21 munis chacun d'une extrémité distale 22, pour la réalisation d'une opération de saisissement.

On notera que, non limitativement, mais préférentiellement, le corps 20 de chacune des branches 2 présente une section transversale en forme de demi-cercle.

Dans ce mode de réalisation, les branches 2 sont actionnées par déformation élastique, tandis que les segments libres 21 consistent en de simples extensions effilées et droites séparées par un espace médian 23, sachant qu'ils pourraient présenter des formes multiples.

Chacun des corps 20 comporte des fentes 24 régulièrement espacées longitudinalement et pratiquées perpendiculairement au plan médian de l'espace 23, et ayant une fonction antidérapante.

Sur la figure 1, ainsi que sur les figures 2 et 3, on peut voir également un système de protection 4, lequel se présente, non limitativement, sous la forme d'un bloc sensiblement cylindrique dans lequel est pratiquée une cavité 40 en forme de gorge longitudinale, délimitée par un fond 41 et deux parois latérales 42 en regard. Une telle gorge peut être ouverte à au moins une extrémité, mais non obligatoirement, afin de faciliter la stérilisation. Toutefois, ladite gorge peut être fermée, la stérilisation s'opérant sous l'effet d'une élévation de température ou bien par l'extrémité opposée par laquelle est introduit le dispositif au sein de son système de protection 4.

A l'une de ses extrémités, la gorge 40 comporte une zone 43 comprenant des moyens de rétention et d'immobilisation dans le sens axial du dispositif de pinces 1.

Ainsi, au niveau de cette zone 43, la partie inférieure 44 de la gorge 40 présente une section transversale oblongue, tandis que sa partie supérieure 45 est d'une largeur légèrement inférieure.

En fait, la largeur de la partie supérieure 45 correspond sensiblement à l'épaisseur du dispositif de pinces 1 au niveau des corps 20 des branches 2 lorsque celles-ci sont rapprochées l'une de l'autre, tandis que les dimensions de la partie inférieure 44 permettent l'ouverture et le non contact des extrémités distales 22.

Ainsi, l'introduction des branches 2 dans la gorge 40, nécessite d'exercer une pression sur les corps 20, pour contraindre les branches 2 à la fermeture, et ainsi franchir la partie supérieure 45, et atteindre la partie inférieure 44 où les branches peuvent s'écarter et se trouver prisonnières.

Afin d'empêcher un déplacement axial du dispositif de pinces 1 dans le système de protection 4, de manière que les extrémité distale 22 demeurent protégées dans la gorge 40, la zone 43 est munie de moyens d'immobilisation qui consistent en des saillies 46 destinées à coopérer avec les fentes 24 des corps 20.

Ces saillies 26 se présentent sous la forme de lames qui s'étendent perpendiculairement à l'axe de la gorge 40, depuis chacune des parois 42 en regard.

On notera que dans ce mode de réalisation, les lames 26 sont également configurées pour immobiliser en rotation le dispositif de pinces 1.

Après mise en place d'un dispositif de pinces 1 dans un système de protection 4, l'ensemble peut subir les opérations de nettoyage et de stérilisation, puis être stocké sans risque d'endommagement des segments libres et des extrémités distales 22 des branches 2 qui demeurent protégées.

Les dimensions de la gorge 40, ainsi que son profil, sont variables, et adaptés aux caractéristiques du dispositif de pinces 1, dont la section, au niveau des corps 20, peut être de forme autre que ronde, carrée ou rectangle par exemple, non limitativement. On notera que dans le cas de section de forme non ronde ou analogue, il n'est pas nécessaire de prévoir des moyens spécifiques d'immobilisation en rotation.

## Revendications

1. Dispositif de pinces (1) et son système de protection (4), du type où ledit dispositif de pinces (1) comporte deux branches (2) mobiles en rapprochement et en éloignement d'un plan axial médian, au travers d'une déformation élastique, ou d'un déplacement de type pivotement, rappelées élastiquement en ouverture, et en sorte que les extrémités distales (22) desdites branches (2) puissent saisir, maintenir, puis relâcher un objet, **caractérisé en ce que** ledit système de protection (4) consiste en un bloc comportant une cavité (40) destinée à loger les extrémités distales (22) desdites branches (2), et **en ce que** ladite cavité (4) et ledit dispositif de pinces (1) comportent d'une part des éléments (20, 44) de formes complémentaires conçus aptes à permettre la rétention par encliquetage dudit dispositif de pinces (1) dans ladite cavité (40), tout en autorisant le maintien desdites branches (2) écartées en sorte que lesdites extrémités distales (22) soient maintenues hors de contact de l'une avec l'autre ; et d'autre part des moyens (46) d'immobilisation dans le sens axial dudit dispositif de pinces (1) par rapport audit système de protection (4).

2. Dispositif selon la revendication 1, **caractérisé en ce que** le système de protection (4) comporte des moyens (46) d'immobilisation en rotation axiale du dispositif de pinces (1).

3. Dispositif selon la revendication 1 ou la revendication 2, **caractérisé en ce que** le système de protection (4) est réalisé dans un matériau supportant des conditions de stérilisation.

4. Dispositif selon la revendication 3, **caractérisé en ce que** le système de protection (4) est réalisé dans un polymère de la famille des polyacétals.

5. Dispositif selon l'une quelconque des revendications 1 à 4, **caractérisé en ce que** la cavité (40) du système de protection consiste en une gorge pratiquée longitudinalement, ouverte à au moins une extrémité, et qui présente, dans le but de rétention, une zone (44) de profil femelle concordant avec le profil d'au moins une partie (20) du dispositif de pinces (1), l'introduction de ladite partie (20) dans ladite zone, nécessitant de traverser une zone de moindre largeur (45) ce qui exige de contraindre les branches (2) en rapprochement.

6. Dispositif selon la revendication 5, **caractérisé en ce que** les empreintes mâles (46) consistent en une ou plusieurs lames qui s'étendent transversalement à l'axe de la gorge, et partiellement dans ladite gorge depuis les parois en regard qui délimitent ladite gorge, tandis les empreintes femelles consistent en au moins une fente (24) pratiquée perpendiculairement au plan axial médian du dispositif de pinces (1).

7. Dispositif selon l'une quelconque des revendications 1 à 6, **caractérisé en ce que** les moyens d'immobilisation dans le sens axial consistent en des empreintes mâles (46) aptes à coopérer avec des empreintes femelles (24) concordantes que comporte le dispositif de pinces (1).
